# EUROPÄISCHE PATENTSCHRIFT

(11) **EP 0 760 633 B1**
(45) Veröffentlichungstag und Bekanntmachung des Hinweises auf die Patenterteilung: **18.08.1999**
(21) Anmeldenummer: 95941713.0
(22) Anmeldetag: 14.12.1995
(51) Int. Cl.: A61B 19/00, A61B 17/86, G01S 5/16

(54) **SYSTEM UND KNOCHENSCHRAUBE ZUR ORTSBESTIMMUNG EINES KÖRPERTEILS**
SYSTEM AND BONE SCREW FOR DETERMINING THE POSITION OF A BODY PART
SYSTEME ET VIS A OS POUR LA DETERMINATION DE LA POSITION D'UNE PARTIE DU CORPS

(30) Priorität: 23.02.1995 DE 19506197
(43) Veröffentlichungstag der Anmeldung: 12.03.1997
(73) Patentinhaber: Aesculap AG & Co. KG, 78532 Tuttlingen (DE)
(72) Erfinder: HASSFELD, Stefan, D-69221 Dossenheim (DE); MÜHLING, Joachim, D-74722 Buchen (DE); LUTZE, Theodor, D-78582 Balgheim (DE); SCHULZ, Joachim, D-78532 Tuttlingen (DE)
(74) Vertreter: Böhme, Ulrich, Dr. Dipl.-Phys.
(86) Internationale Anmeldenummer: EP9504954
(87) Internationale Veröffentlichungsnummer: WO9625893

(56) Entgegenhaltungen:
- EP-A- 0 413 588
- EP-A- 0 429 148
- WO-A-94/17733
- US-A- 5 230 623
- US-A- 5 249 581

## Beschreibung

Die Erfindung betrifft ein Ortsbestimmungssystem für ein Körperteil mit mehreren mit Kabeln versehenen Strahlungssendern bzw. Strahlungsempfängern und mit ortsfesten Empfängern bzw. Sendern sowie mit einer Datenverarbeitungseinrichtung, die aus der Richtung und/oder der Laufzeit der übertragenen Strahlung und/oder der Stärke oder Frequenz des Strahlungsfeldes die Positionen der Strahlungssender bzw. Strahlungsempfänger bestimmt.

Außerdem betrifft die Erfindung eine Knochenschraube.

Bei medizinischen Operationen, insbesondere bei Kopfoperationen, ist es üblich, vor der Operation beispielsweise durch ein Computertomogramm genaue Daten über die internen Körperstrukturen zusammenzutragen und diese dann bei der Operation zur Verfügung zu stellen, beispielsweise durch Gehirn-Schnittbilder auf einem Monitor. Um hier eine genaue räumliche Zuordnung zu ermöglichen, werden bei den präoperativen Untersuchungen Markierungselemente unverschieblich an einem Körperteil festgelegt, sogenannte fiducials. Es handelt sich dabei beispielsweise um Knochenschrauben, die in den Schädel eingedreht sind und die geringfügig über die Kopfhaut vorstehen. Bei der Aufnahme der Schichtbilder beispielsweise durch Computertomogramme oder Kernspintomogramme sind diese Markierungselemente sichtbar, so daß durch diese Markierungselemente beispielsweise die relative Lage einer aufgenommenen Schnittebene zu dem untersuchten Kopf überprüfbar ist.

Bei der Operation selber verbleiben diese Markierelemente im Körper. Nachdem der Körperteil zu Beginn der Operation in eine gewünschte Lage gebracht wurde, können anhand dieser Markierelemente die aufgenommenen Schichtbilder mit dem betreffenden Körperteil in Übereinstimmung (korreliert) gebracht werden.

Während der Operation sind allerdings Bewegungen des Körperteils nicht auszuschließen, und dann stimmt die Zuordnung der Momentanposition des Körperteils zu dem betreffenden Schichtbild nicht mehr, also beispielsweise zu einer Behandlungseinrichtung.

Man versucht daher den Körperteil in Halterahmen festzulegen und zu fixieren. Dies ist jedoch nicht immer möglich, da bei der Operation häufig eine Bewegung des Körperteils vorgenommen werden muß.

Gattungsbildende Systeme und Knochenschrauben sind aus der US-A-5249581 bekannt. Diese arbeiten mit Strahlungssendern oder Strahlungsempfängern, die mittels Schaft und Halterahmen oder Knochenschraube an einem Körperteil angebracht werden.

In der US-A-5,230,623 ist ein weiteres Markierungssystem beschrieben, bei dem Markierungen am Körper durch den Operateur physisch durch Berührung mit einem Pointer festgestellt werden können. Es können auch anatomische Punkte als Markierungen verwendet werden, beispielsweise die Nase oder Strukturen in der Umgebung des Ohrs oder des Auges. Weitere Angaben über die Natur dieser Markierungspunkte sind dieser Druckschrift nicht zu entnehmen.

In der WO 94/17733 sind Markierungselemente angegeben, die für tomographische Verfahren oder für dimagnetische Resonanz bestimmt sind, bei denen eine Substanz in einem Behälter plaziert wird. Diese Substanz kann dann durch Röntgenstrahlen oder durch die Absorption von Radiowellen (Kernspinresonanz) nachgewiesen werden.

Ausgehend von einem Stand der Technik, wie er durch die US-A-5,249,581 beschrieben wird, liegt der Erfindung die Aufgabe zugrunde, ein gattungsgemäßes Ortsbestimmungssystem so zu verbessern, daß die Strahlungssender und Strahlungsempfänger in einfachster Weise am Körper plaziert werden können.

Diese Aufgabe wird bei einem Ortsbestimmungssystem der eingangs beschriebenen Art erfindungsgemäß dadurch gelöst, daß der Strahlungssender oder Strahlungsempfänger in eine Knochenschraube herausziehbar eingesteckt ist.

Dadurch wird der Strahlungsempfänger oder Strahlungssender nicht mehr in relativ großer Entfernung von dem zu überwachenden Körperteil an einem Rahmen oder einer Klemme festgelegt, sondern direkt an den Knochenschrauben, die in den Körperteil eingeschraubt sind und die somit relativ zu dem Körperteil mit der erforderlichen Sicherheit unbeweglich sind. Dadurch ist sichergestellt, daß die Strahlungssender bzw. Strahlungsempfänger relativ zu dem überwachten Körperteil während der gesamten Operation zuverlässig ihre Relativposition einhalten. Insgesamt ergibt sich dabei eine wesentlich erhöhte Genauigkeit der Positionsüberwachung, außerdem läßt sich diese Positionierung auch dann verwenden, wenn bei bestimmten Operationen auf die Verwendung eines an den Körperteil angelegten Rahmens oder eine Klemme verzichtet wird. Es können zur Festlegung der Strahlungsempfänger bzw. Strahlungssender die ohnehin in den Körperteil eingedrehten Knochenschrauben verwendet werden, die als Markierelemente für die Korrelation von Körperteil und Schichtbildern dienen.

Durch das Einstecken der Strahlungsempfänger oder Strahlungssender in die Knochenschraube können diese auch einfach durch Herausziehen entfernt werden, so daß der Patient nach der Operation mit verbleibenden Knochenschrauben versorgt werden kann, beispielsweise können diese Knochenschrauben bei einer nachfolgenden Untersuchung von Bedeutung sein, etwa bei der Aufnahme neuer Schichtbildaufnahmen.

Günstig ist es dabei, wenn man als Strahlungssender eine Leuchtdiode verwendet. Diese sendet Strahlung aus, die von ortsfesten Strahlungsempfängern empfangen werden kann. Selbstverständlich könnte an den Knochenschrauben auch ein Strahlungsempfänger für eine sichtbare Strahlung angeordnet sein, jedoch ist es vorteilhaft, den Strahlungssender an der Knochenschraube festzulegen, da üblicherweise die Strahlungssender kleiner sind als die Strahlungsempfänger.

Auch andere physikalische Sender/Empfänger-Kombinationen sind denkbar, beispielsweise Ultraschallsender und Ultraschallempfänger oder Strahlungssender und Strahlungsempfänger im nicht sichtbaren Bereich, Strahlungsempfänger oder Spulen, die elektromagnetische Strahlung empfangen und senden.

Günstig ist es, wenn man Knochenschrauben verwendet, die nur wenige Millimeter über die Oberfläche des Körperteils vorstehen. Dadurch ergeben sich keine großen Hebelarme zwischen Körperteil und Strahlungssender bzw. Strahlungsempfänger, d. h. die Relativpositionen des Strahlungssenders bzw. des Strahlungsempfängers relativ zum Körperteil ist in diesem Falle praktisch unverändert.

Üblicherweise wird man eine größere Anzahl von Knochenschrauben mit Strahlungssender bzw. mit Strahlungsempfänger verwenden, um auf diese Weise verschiedene Freiheitsgrade der Körperteilbewegung bestimmen zu können. Günstig ist es dabei, wenn man die Relativpositionierung dieser Knochenschrauben untereinander überwacht und wenn man die Positionsdaten eines Strahlungssenders bzw. Strahlungsempfängers bei der Positionsbestimmung des Körperteils unberücksichtigt läßt, dessen Position sich gegenüber der Position der übrigen Strahlungssender bzw. Strahlungsempfänger geändert hat. Wenn bei einer größeren Anzahl von am Körperteil festgelegten Strahlungssendern oder Strahlungsempfängern einer dieser Strahlungssender bzw. Strahlungsempfänger gegenüber allen anderen eine geänderte Position zeigt, so ist dies ein Indiz dafür daß sich dieser Strahlungssender bzw. Strahlungsempfänger ungewollt verschoben hat, sei es dadurch, daß er sich von der Knochenschraube gelöst hat, sei es dadurch, daß bei der Operation der die Knochenschraube tragende Knochen selbst beschädigt worden ist. Sobald dies auftritt, kann das Datenverarbeitungssystem selbständig die Daten dieses geänderten Strahlungssenders bzw. Strahlungsempfängers ausscheiden und die Positionierung des Körperteils nur aufgrund der Positionsdaten der übrigen Strahlungssender bzw. Strahlungsempfänger bestimmen, so daß diese unbeabsichtigte Abweichung nicht in das Positionsbestimmungsergebnis eingeht. Außerdem kann bei Bedarf ein Warnsignal gegeben werden, das auf die Verschiebung eines Strahlungssenders bzw. Strahlungsempfängers hinweist.

Die Erfindung bezieht sich auch auf eine Knochenschraube, die dadurch gekennzeichnet ist, daß sie einen mit einem Kabel versehenen Strahlungssender oder Strahlungsempfänger trägt, der in die Knochenschraube herausziehbar eingesteckt ist.

Die nachfolgende Beschreibung einer bevorzugten Ausführungsform der Erfindung dient im Zusammenhang mit der Zeichnung der näheren Erläuterung. Es zeigen:
- Figur 1:: eine schematische Ansicht eines Lagebestimmungssystems und
- Figur 2:: eine Teilschnittansicht einer Knochenschraube mit eingesetzter Leuchtdiode als Strahlungssender.

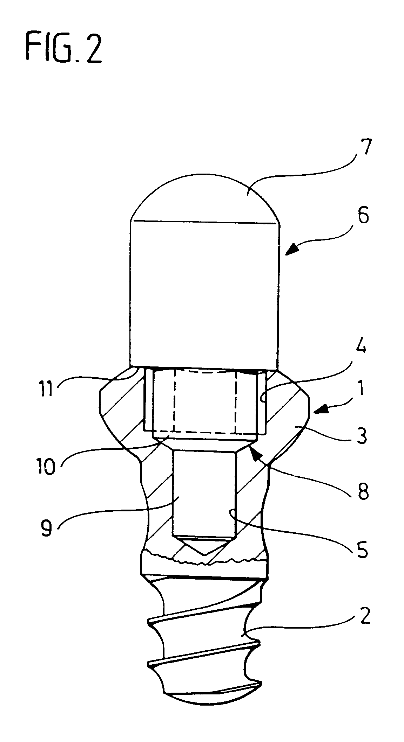

In Figur 2 ist eine übliche Knochenschraube 1 mit einem Gewinde 2, einem Kopf 3 und einer im Kopf angeordneten, sechseckigen Einstecköffnung 4 für ein Eindrehwerkzeug dargestellt. An die Einstecköffnung 4 schließt sich an deren Boden konzentrisch zur Einstecköffnung 4 eine zylindrische Sacklochbohrung 5 an.

In diese Knochenschraube 1 ist eine Leuchtdiode 6 eingesetzt, die einen Diodenkopf 7 sowie einen Einsteckfuß 8 umfaßt. Dieser greift mit einem Einsteckstift 9 in die Sacklochbohrung 5 ein und wird dort im Klemmsitz gehalten. Eine Erweiterung 10 des Einsteckfußes 8 ist dabei in der Einstecköffnung 4 der Knochenschraube 1 aufgenommen.

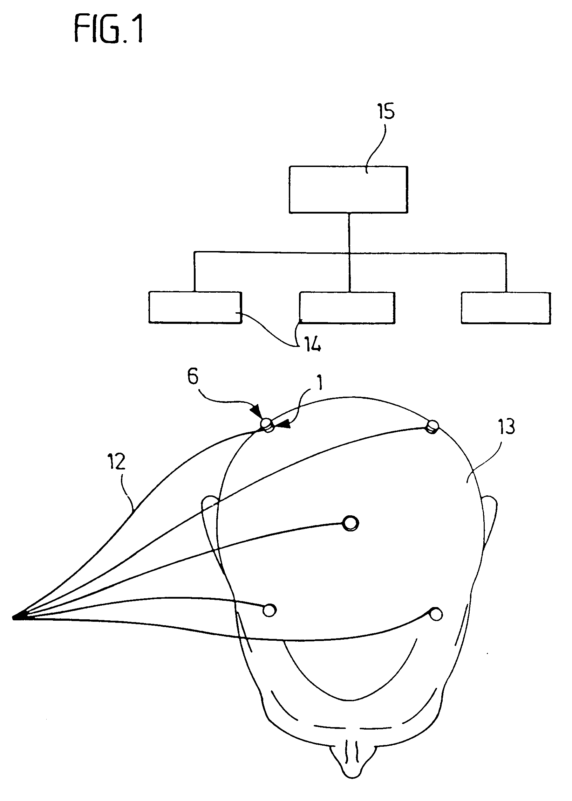

Der Diodenkopf 7 stützt sich mit seiner Unterseite 11 an der oberen Kante des Kopfes 3 ab und nimmt somit im eingesetzten Zustand gegenüber der Knochenschraube 1 eine genau definierte Lage ein. In aus Figur 2 nicht ersichtlicher Weise wird die Leuchtdiode 6 über ein Kabel mit einer Energiequelle verbunden, so daß die Leuchtdiode 6 nach Bedarf zum Leuchten gebracht werden kann. Ein solches Kabel 12 ist in der Darstellung der Figur 1 ersichtlich.

Dort ist weiterhin gezeigt, wie mehrere Knochenschrauben 1 in einem beliebigen Muster an einem Körperteil festgelegt werden können, im dargestellten Beispiel am Kopf 13 eines Patienten. Bei einer präoperativen Untersuchung werden die Knochenschrauben 1 in diesem gewünschten Muster in den Schädelknochen des Patienten eingedreht und bilden dort in der Umgebung der geplanten Eingriffsstelle ein Muster von Markierelementen oder fiducials, die bei der Aufnahme von Computertomogrammen mit erfaßt werden, so daß bei der Erstellung von Schnittbildaufnahmen deren relative Lage zum Kopf 13 des Patienten bestimmbar ist.

Bei einer nachfolgenden Operation werden in jede Knochenschraube 1 Leuchtdioden 6 eingesetzt, die dann über Kabel 12 mit einer gemeinsamen Energie- und Steuerquelle verbunden werden. Die von den Leuchtdioden 6 ausgesandte Strahlung trifft auf mehrere ortsfeste Strahlungsempfänger 14, und zwar durch deren unterschiedliche Anordnung mit unterschiedlichem Auftreffwinkel und gegebenenfalls auch nach einer unterschiedlichen Laufzeit. Diese Daten werden von den Strahlungsempfängern 14 einer Datenverarbeitungsanlage 15 zugeführt, die aufgrund dieser unterschiedlichen Daten die relative Lage jeder Leuchtdiode 6 relativ zu den Strahlungsempfängern 14 bestimmt. Durch die Lagebestimmung sämtlicher Leuchtdioden 6 ergibt sich eine vollständige Lage- und Orientierungsbestimmung des Kopfes 13. Diese Lage- und Orientierungsbestimmung kann während der gesamten Operation laufend wiederholt werden, so daß auch bei einer Bewegung des Kopfes 13 relativ zu den Strahlungsempfängern 14 die relative Lage jederzeit zu bestimmen ist.

Sollte sich die Position einer Leuchtdiode 6 relativ zur Position der anderen Leuchtdioden ändern, so wird dies ebenfalls von der Datenverarbeitungsanlage festgestellt, und dies ist dann ein Zeichen dafür, daß unvorhergesehen entweder die entsprechende Leuchtdiode 6 nicht mehr richtig in der Zugeordneten Knochenschraube 1 gehalten ist oder sogar diese Knochenschraube selbst nicht mehr in der vorgesehenen Weise am Kopf 13 fixiert ist. Die Positionsdaten dieser Knochenschraube 1 und dieser Leuchtdiode 6 werden von der Datenverarbeitungsanlage 15 in einem solchen Falle ignoriert und nicht zur Bestimmung der Lage und Orientierung des Kopfes verwendet, so daß keine Fehlbestimmungen eintreten können. Außerdem kann gegebenenfalls ein Warnungssignal erzeugt werden, das den Operateur auf eine Kontrolle dieser fehlpositionierten Leuchtdiode hinweist.

Die Leuchtdioden 6 können von den Knochenschrauben 1 einfach durch Herausziehen entfernt werden, so daß der Patient nach der Operation mit verbleibenden Knochenschrauben 1 versorgt werden kann, beispielsweise können diese Knochenschrauben 1 bei einer nachfolgenden Untersuchung von Bedeutung sein, etwa bei der Aufnahme neuer Schichtbildaufnahmen.

## Patentansprüche

1. Ortsbestimmungssystem für ein Körperteil mit mehreren mit Kabeln (12) versehenen Strahlungssendern (6) bzw. Strahlungsempfängern und mit mehreren ortsfesten Empfängern (14) bzw. Sendern sowie mit einer Datenverarbeitungseinrichtung (15), die aus der Richtung und/ oder der Laufzeit der übertragenen Strahlung und/ oder der Stärke oder Frequenz des Strahlungsfeldes die Positionen der Strahlungssender bzw. Strahlungsempfänger bestimmt, dadurch gekennzeichnet, daß jeweils ein Strahlungssender (6) oder Strahlungsempfänger herausziehbar in eine Knochenschraube (1) eingesteckt ist.

2. Ortsbestimmungssystem nach Anspruch 1, dadurch gekennzeichnet, daß der Strahlungssender (6) eine Leuchtdiode ist.

3. Knochenschraube, dadurch gekennzeichnet, daß ein mit einem Kabel (12) versehener Strahlungssender (6) oder Strahlungsempfänger in die Knochenschraube (1) herausziehbar eingesteckt ist.

4. Knochenschraube nach Anspruch 3, dadurch gekennzeichnet, daß der Strahlungssender (6) eine Leuchtdiode ist.

## Claims

1. A system for determining the location of a body part, comprising a plurality of radiation transmitters (6) or radiation receivers provided with cables (12), a plurality of fixed receivers (14) or transmitters, and a data processing installation (15) which determines the positions of the radiation transmitters or radiation receivers on the basis of the direction and/or the transit time of the transmitted radiation and/or the strength or frequency of the radiation field, characterised in that a radiation transmitter (6) or radiation receiver is removably inserted into a bone screw (1).

2. A locating system according to claim 1, characterised in that the radiation transmitter (6) is a light-emitting diode.

3. A bone screw, characterised in that a radiation transmitter (6) or radiation receiver provided with a cable (12) is removably inserted into the bone screw (1).

4. A bone screw according to claim 3, characterised in that the radiation transmitter (6) is a light-emitting diode.

## Revendications

1. Système de détermination de position pour une partie du corps avec des émetteurs de rayonnement (6) ou des récepteurs de rayonnement munis de câbles (12) et avec plusieurs récepteurs (14) ou émetteurs fixes ainsi qu'avec un dispositif de traitement des données (15) qui détermine les positions des émetteurs de rayonnement ou des récepteurs de rayonnement d'après la direction et/ou la durée de propagation du rayonnement transmis et'ou l'intensité ou la fréquence du champ de rayonnement, caractérisé en ce que, dans chaque cas, un émetteur de rayonnement (6) ou un récepteur de rayonnement est inséré de manière amovible dans une vis à os (1).

2. Système de détermination de position selon la revendication 1, caractérisé en ce que l'émetteur de rayonnement (6) est une diode luminescente.

3. Vis à os caractérisée en ce qu'un émetteur de rayonnement (6) ou un récepteur de rayonnement muni d'un câble (12) est inséré de manière amovible dans la vis à os (1).

4. Vis à os selon la revendication 3, caractérisée en ce que l'émetteur de rayonnement (6) est une diode luminescente.
